# EUROPÄISCHE PATENTSCHRIFT

(11) **EP 0 612 750 B1**
(45) Veröffentlichungstag und Bekanntmachung des Hinweises auf die Patenterteilung: **03.03.1999**
(21) Anmeldenummer: 94105662.4
(22) Anmeldetag: 21.11.1985
(51) Int. Cl.: C07D 501/18, C07F 7/18, C07D 501/06

(54) **Cephalosporinderivative**
Cephalosporinderivatives
Dérivés de céphalosporine

(30) Priorität: 23.11.1984 AT 3715/84; 23.11.1984 AT 3716/84
(43) Veröffentlichungstag der Anmeldung: 31.08.1994
(62) Teilanmeldung aus: 86900093.5
(73) Patentinhaber: BIOCHEMIE Gesellschaft m.b.H., 6250 Kundl Tirol (AT)
(72) Erfinder: Ascher, Gerd, A-6250 Kundl (AT); Thaler, Heinrich, Deceased (AT); Ludescher, Johannses, A-6250 Breitenbach (AT)
(74) Vertreter: Wymann, Gérard

(56) Entgegenhaltungen:
- EP-A- 0 124 889
- EP-A- 0 125 576
- EP-A- 0 154 417
- FR-A- 2 457 295

## Beschreibung

Cephalosporine sind wertvolle in der Humanmedizin verwendete Antibiotika. Ein Teil dieser Verbindungen (z. B. Cefalotin, Cefotaxim) leitet sich von dem Grundkörper 7-Aminocephalosporansäure (7-ACA) ab. 7-ACA kann durch Fermentation von Cephalosporin C, anschließende Isolierung des Naturprodukts und Seitenkettenabspaltung einfach hergestellt werden und ist ein leicht erhältliches Handelsprodukt.

Ein anderer, wesentlich größerer Teil der heute therapeutisch verwendeten Cephalosporine leitet sich hingegen von 7-Aminocephalosporansäuren ab, die einen anderen Rest als 7-ACA in 3-Stellung tragen (z. B. Cefaloridin, Cefamandol, Cefazolin, Cefoperazon, Ceftazidime, Ceftriaxon, Cefmenoxim, Cefonicid). Für die Herstellung von derartigen Verbindungen muß üblicherweise zunächst bei 7-ACA ein Austausch des Substituenten in 3-Stellung durchgeführt und die 3-substituierte 7-ACA isoliert werden. Ein weiterer Schritt führt dann zur Acylierung in 7-Stellung und zur Isolierung des gewünschten Cephalosporinantibiotikums.

Die Reaktion kann auch umgekehrt durchgeführt werden, das heißt, daß zuerst 7-ACA mit dem gewünschten Rest in 7-Stellung acyliert und anschließend das Acylderivat der 7-ACA isoliert wird. Nachfolgender Austausch in wäßrigen Puffersystemen führt dann üblicherweise zum gewünschten Cephalosporin-Endprodukt.

Beide Reaktionen haben Begrenzungen, das heißt, sie lassen sich nicht wahllos mit jeder Kombination von gewünschten Resten in 3- oder 7-Stellung durchführen. In allen Fällen muß über Acylierung/Austausch und/oder Isolierung im protischen Medium gearbeitet werden. Enthält die Seitenkette in 7-Stellung selbst eine acylierbare oder aktivierbare Funktion, so muß diese Gruppe zuerst mit einer ausgewählten Schutzgruppe geschützt und nach der Isolierung des gewünschten Cephalosporin-Endprodukts die Schutzgruppen wieder abgespalten werden.

Der Stand der Technik zur Herstellung von derartigen Verbindungen weist im wesentlichen drei Verfahren auf, die jedoch alle mit gewissen Nachteilen behaftet sind. So beschreibt die DE-OS 2,804.896 (TOYAMA) die Umsetzung von 7-Aminocephem(3)-4-carbonsäuren mit Lewis-Säuren bzw. mit Protonensäuren im Überschuß, wobei vorzugsweise Bortrifluorid verwendet wird. Es ist bekannt, daß derartige Reagenzien äußerst toxisch sind, Umweltprobleme verursachen und außerdem β-Lactame unter stark sauren Bedingungen im protischen Medium instabil sind.

Ein zweites Verfahren beschreibt die Austauschreaktion in 3-Stellung in wäßrigen Puffersystemen (US-PS 3,516.997). Dieses Verfahren gibt geringe Ausbeuten, da unter den verwendeten Bedingungen (7-ACA + heterocyclisches Thiol) Cephalosporine in Pufferlösungen zwischen pH 5 und 9 zu einem erheblichen Teil zersetzt und daher die Titelverbindungen in ungenügender Reinheit erhalten werden.

Ein drittes Verfahren beschreibt die Acylgruppen-Abspaltung der Seitenkette in 7-Position bei einem Cephalosporin, das schon den richtigen Substituenten in 3-Stellung trägt (z.B. US-PS 4,369.313). Zur Herstellung der In diesem Patent verwendeten Ausgangsmaterialien muß jedoch 7-ACA zunlichst in 7-Stellung acyliert, dann der Austausch in 3-Stellung vorgenommem und zum Schluß die Seitenkette in Position 7 abgespalten werden. Gesamthaft gesehen ist diese Reaktionsfolge von der Ausbeute ab 7-ACA als unwirtschaftlich zu betrachten.

In der DE-A-3 316 798, Hoechst AG, ist u. a. ein Verfahren zur Herstellung von in 3-Stellung durch eine Base substituierte 7-Aminocephalosporansäure durch Zugabe der entsprechenden Base, gefolgt von Trimethyljodsilan zu der 7-Aminocephalosporansäure in einein Verdünnungsmittel und Erhitzen auf Rückfluss, oder durch Erhitzen der Base mit dem Trimethyljodsilan in einem Verdünnungsmittel auf Rückfluss und Zugabe der 7-Aminocephalosporansäure und weiteres Erhitzen auf Rückfluss beschrieben. Die Ausbeuten nach diesem Verfahren sind aber in vielen Fällen unbefriedigend.

In EP-A-0 154 417, Yamanouchi Pharmaceutical Co. Ltd., die bezüglich der vorliegenden Anmeldung für die Vertragsstaaten Deutschland, Frankreich, Grossbritannien und Italien eine Veröffentlichung gemäss Art. 54(3) EPÜ darstellt, ist geoffenbart, dass 7-Aminocephalosporansäure erst in 3-Stellung jodiert und dann mit Hilfe eines Silylierungsmittels in 7- und 3-Stellung silyliert werden könne. Gemäss der Beschreibung wird angenommen, dass dabei 7-Trimethylamino-3-jodomethylcephem(3)-4-carbonsäuretrimethylsilylester als Zwischenprodukt entstehen soll. Wie sich herausgestellt hat, werden aber bei dieser Art der Reaktionsführung Gemische der mono- und bissilylierten Verbindung erhalten, die praktisch untrennbar sind und die die bisilylierte Verbindung nur zu etwa 64Gew% enthalten.

Es wurde nun unerwarteterweise gefunden, dass ein reiner 7-Trialkylamino-3-jodomethylcephem(3)-4-carbonsäuretrialkylsilylester ein besonders geeignetes Zwischenprodukt für die Herstellung von 7-Aminocephalosporansäuren, die in 3-Stellung durch eine Base substituiert sind, ist, dass eine hohe Ausbeute bei der Substitionsreaktion des Jodatoms von 7-Trimethylamino-3-jodomethylcephem(3)-4-carbonsäuretrimethylsilylester in 3-Stellung durch eine Base ganz wesentlich von die Reinheit des Esters abhängt und dass 7-Trialkylamino-3-jodo-methylcephem(3)-4-carbonsäuretrialkylsilylester durch Jodierung von 7-Trialkylsilylaminocephalosporansäure-trialkylsilylester mit Trialkyljodsilan in hoher Reinheit herstellbar sind.

Im vorliegenden Verfahren wird 7-ACA oder ein Derivat davon in ihr N,O-bissilyliertes Derivat überführt, hierauf mit einem Trialkyljodsilan zur 3-Jodmethylverbindung umgesetzt, durch Zugabe eines nucleophilen Reaktionspartners in das gewünschte 3-substituierte Derivat der N,O-bissilylierten 7-ACA übergeführt, dieses entweder entschützt oder mit einem Acylierungsmittel, das die gewünschte Acylgruppe in 7-Stellung einführen soll, versetzt und die Reaktion durch Entschützen zu Ende geführt.
Das volle Verfahren kann, wenn erwünscht, ohne Isolierung der einzelnen Zwischenprodukte durchgeführt werden.

Die vorliegende Erfindung betrifft das neue Zwischenprodukt 7-Trimethylamino-3-jodomethylcephem(3)-4-carbonsäuretrimethylsilylester der Formel III, die Verwendung eines Zwischenproduktes der Formel III zur Herstellung eines Cephalosporinderivates, wobei das Zwischenprodukt gegebenenfalls aus einer Verbindung der Formel IV, worin R, R₁ und R₂ die unten angegebene Bedeutung haben, durch Umsetzung mit Trialkyljodsilan, z.B. Trimethyljodsilan, beispielsweise unter Verwendung von 1,1 bis 1,5 Äquivalenten Trialkyljodsilan per Äquivlent Cephalosporinderivat der Formel IV, hergestellt ist; und ein Verfahren zur Herstellung eines Cephaloporinderivates der Formel I, worin R₁, R₃, R₄ und R₅ die unten angegebenen Bedeutung haben, das dadurch gekennzeichnet ist, dass , dass ein Cephalosporinderivat der Formel III mit einem gewünschten Nucleophil umgesetzt wird und das dabei erhaltene Derivat, das in 3-Stellung durch den Rest des Nucleophils substituiert ist, entweder durch Abspaltung der Silylschutzgruppen entschützt oder mit einem Acylierungsmittel in 7-Stellung acyliert und durch Abspaltung der Silylschutzgruppen entschützt wird; wobei das Cephalosporinderivat der Formel III gegebenenfalls aus einer Verbindung der Formel IV, worin R, R₁ und R₂ die obgenannte Bedeutung haben, durch Umsetzung mit Trialkyljodsilan, z.B. Trimethyljodsilan, beispielsweise unter Verwendung von 1,1 bis 1,5 Äquivalenten Trialkyljodsilan per Äquivlent Cephalosporinderivat der Formel IV, hergestellt ist.

Das Verfahren kann durch folgendes allgemeines Reaktionsschema beschrieben werden: 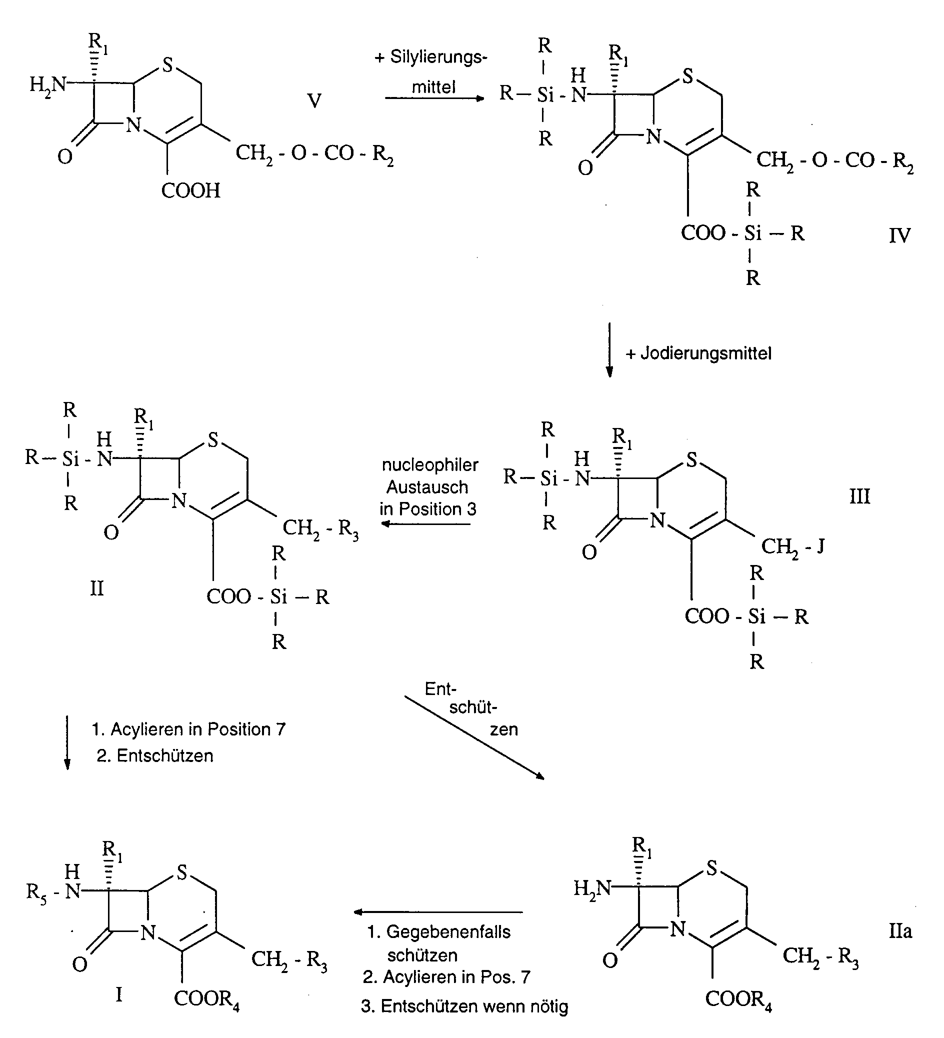 In den Formeln 1 bis V stehen R₁ für Wasserstoff oder die Methoxygruppe, R₂ für die Methyl- oder Aminogruppe, R für eine niedere Alkylgruppe und R₄ für Wasserstoff, Alkali oder eine negative Ladung bzw. OR₄ für Halogen. R₅ bedeutet eine in der Cephalosporinchemie übliche Acylgruppe. R₃ für den Rest eines Nukleophils steht. Die Erfindung betrifft insbesondere die Stufen III to II; sowie die Reihenfolgen III zu II zu IIa; III to II/IIa→I.

Dieses Verfahren kann beispielsweise folgendermaßen ausgeführt werden:

Eine 7-Aminocephem(3)-4-carbonsäure der Formel V, vorzugsweise 7-ACA, wird in einem unter den Reaktionsbedingungen inerten Losungsmittel suspendiert und in Gegenwart eines Überschusses an Silylierungsmittel rückflußerhitzt. Als Lösungsmittel für diese Silylierungsreaktion kommen halogenierte Kohlenwasserstoffe in Frage, wie beispielsweise Dichlormethan, Chloroform, 1,2-Dichlorethan, 1,1,2-Trichlorethan und Tetrachlorethylen, organische Nitrile, wie Acetonitril, Propionitril, Nitroalkane, wie Nitromethan oder Sulfone, wie Sulfolan. Die Silylierungsreaktion kann entweder bei hochsiedenden Lösungsmitteln durch Erhitzen auf die entsprechenden Temperaturen oder bei niedriger siedenden Lösungsmit teln durch eine spezielle Katalyse einer organischen Sauerstoffsaure, z.B. Quadratsäure, Trifluoressigsäure oder deren Gemische, durchgeführt werden. Als Silylierungsmittel bei dieser Reaktion können übliche Silylierungsmittel wie Hexamethyldisilazan, Trimethylchlorsilan, Bistrimethylsilylharnstoff, N-Trimethylsilyldialkylamine oder bis- oder monosilylierte Acetamide bzw. Gemische dieser Reagenzien, verwendet werden. Es ist bevorzugt, dass die Bissilylierung zu 100% verläuft, da geringe Mengen an Monosilylderivat die weitere Umsetzung erheblich stören können. Die Umsetzung der so erhaltenen Verbindungen der Formel IV wird beispielsweise so vorgenommen, dass zu dem erhaltenen Reaktionsgemisch 1,1 bis 1,5 Äquivalente Trialkyljodsilan, z.B. Trimethyljodsilan zugegeben werden und die Reaktion bei Temperaturen von -20 bis +50°C, vorzugsweise bei 0 bis 45°C, durchgeführt wird. Es ist überraschend, dass eine Verbindung der Struktur von 3-Jodmethyl-N,O-bistrialkylsilyl-7-aminocephalosporansäure stabil ist. Bei einigen ähnlichen Strukturen, die in der Literatur beschrieben werden, erfolgt exzessive Selbstalkylierung im Stickstoff (DE-OS 3,212.900, YAMANOUCHI). Die Tatsache, dass die erfindungsgemässen N,O-Bis-trialkylsilyl-3-jodmethyl-7-aminocephalosporansäuren stabil sind, kann der sterischen Hinderung der Trialkylsilylgruppe am Stickstoff zugeschrieben werden. Es ist deshalb essentiell, reine Bissilylverbindung vorliegen zu haben, da das Vorliegen von Monosilylverbindungen zu exzessiver Zersetzung und Polymerisation führt.

Bistrimethylsilyl-3-jodmethyl-7-aminocephem(3)-4-carbonsäuren der Formel III können durch Entfernen des überschüssigen Lösungsmittels und Trialkyljodsilans im Vakuum in reiner Form isoliert und mittels des NMR-Spektrums charakterisiert werden.

Die Cephalosporinderivate der Formel III können leicht in 3-substituierte 7-Aminocephem(3)-4-carbonsäuren der Formel II bzw. IIa überführt werden, wobei die Verbindungen der Formel III nicht isoliert werden müssen, und zwar durch Umsetzung mit einem Nukleophile wie z .B.
a) einem tertiären (Stickstoff) Heterocyclus (z.B. Pyridin, Chinolin) oder einem tertiären aliphatischen oder cycloaliphatischen Amin, z.B. N-Ethylpiperidin oder N-Methylpyrrolidin, oder
b) einem Thiolatanion eines mercaptosubstituierten Heterocyclus oder dem freien Mercaptoheterocyclus und einem Säurefängor, oder
c) einem einen sekundären Stickstoff tragenden Heterocyclus, und gegebenenfalls gleichzeitiger oder anschließender Entschützung.

Speziell kommen als solche Reaktionspartner für die Verbindungen der Formel III in Frage:
a) aromatische Stickstoffbasen wie Pyridin, Chinolin, die durch einen oder mehrere Substituenten zusätzlich substituiert sein können; aliphatische Trialkylamine, wobei Alkyl vorzugsweise die Bedeutung von C₁ - C₄ hat, und carbocyclische, gesättigte oder ungesättigte tertiäre Amine, z.B. N-Ethylpiperidin, N-Methylpyrrolidin, Chinuclidin u.a.m.,
b) Salze von mercaptosubstituierten Heterocyclen bzw. die freien Mercaptoverbindungen in Gegenwart von Säurefängern,
c) Heterocyclen, die einen sekundären alkylierten Stickstoff haben, z.B. Triazole, Tetrazole, Pyrazole, Pyrrolidine, Imidazole.

Diese Umsetzungen mit obigen Reaktionspartnern können in den obgenannten Lösungsmitteln bei Temperaturen von -78 bis +70° C, vorzugsweise -50 bis +40° C, durchgeführt werden. Bei schwerer Löslichkeit eines-Reaktionspartners in obgenannten Lösungsmitteln kann nach Bedarf ein absolut trockenes aprotisches polares Lösungsmittel, z.B. DMF, HMPT, zugesetzt werden.

Anschließend wird zur Herstellung der Verbindungen der Formel I im gleichen Reaktionsgemisch nach an sich bekannten Methoden die gewünschte Acylgruppe in Position 7 eingeführt und, falls vorhanden, die Silylgruppe abgespalten.

Für die Acylierung werden vorzugsweise Verbindungen der Formel II bzw. IIa in der Reaktionsmischung hergestellt, worin R₁ obige Bedeutung besitzt und R₃
a) eine aromatische Stickstoffbase der Formel 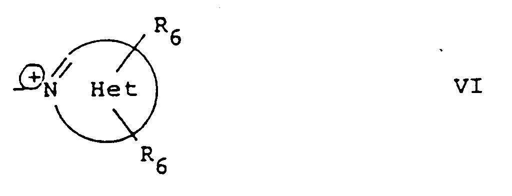 wobei Het Pyridin, Chinolin, Pyrimidin oder Thiazol und R₆ Carboxy, Carbamido, einen Sulfonsäurerest oder einen in der β-Lactamchemie üblichen Substituenten bedeuten, oder beide R₆ einen gegebenenfalls substituierten carbocyclischen Ring bilden, oder
b) eine aliphatische oder cycloaliphatische Stickstoffbase der Formel 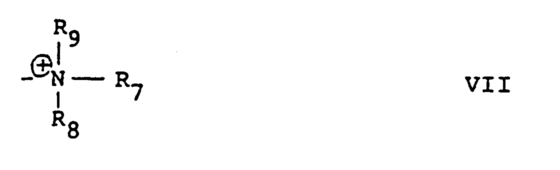 wobei die Substituenten R₇, R₈ und R₉ gleich oder verschieden sein können und jeweils eine niedere Alkyl- oder niedere Alkenylgruppe bedeuten oder R₇ mit R₈ und dem Stickstoffatom einen durch R₉ alkylsubstituierten fünf- oder sechsgliedrigen carbocyclischen gesättigten oder ungesättigten Ring bedeuten, wobei R₉ zusätzlich eine 1,3- oder 1,4-Alkylen- oder Vinylenbrücke darstellen kann, oder
c) einen gegebenenfalls substituierten heterocyclischen Thiolrest der Formel 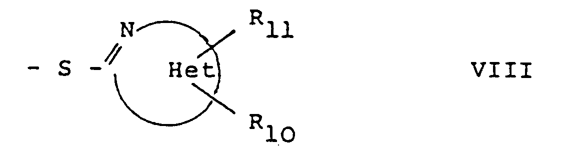 in dem Het einen fünf- oder sechsgliedrigen Heterocyclus bedeutet und R₁₀ und R₁₁ für Alkyl, Halogen oder Alkenyl steht, wobei ein Alkylrest wieder durch Sulfonsäuregruppen, Acylamino, Dialkylamino, Oxo oder Hydroxy substituiert sein kann, oder
d) einen Rest der Formel 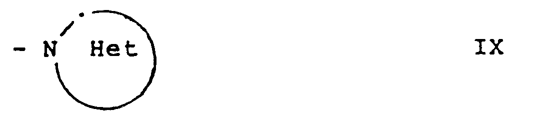 wobei Het Tetrazol, Triazol, Imidazol, Pyrrolidin oder Pyrazol, die durch eine niedere Alkylgruppe substituiert sein können, bedeutet. Diese Verbindungen können durch Zusatz einer Verbindung der Formeln 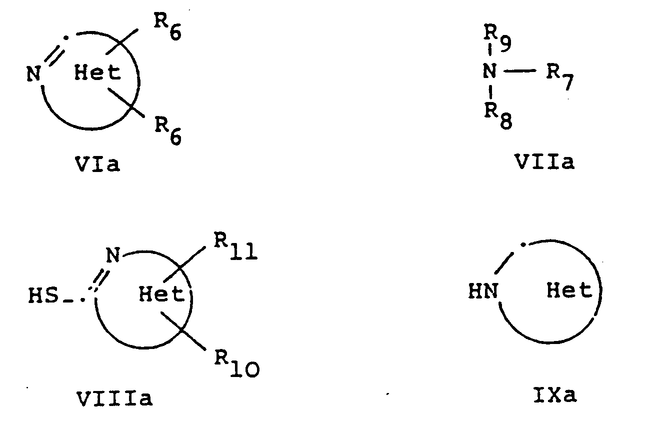 worin R₆ bis R₁₁ und Het obige Bedeutung besitzen, zum Reaktionsgemisch, das eine Verbindung der Formel II bzw. IIa enthält, erhalten werden.

In den nachfolgenden Beispielen, die die Erfindung erläutern sollen, ohne jedoch ihren Umfang einzuschränken, erfolgen alle Temperaturangaben in Celsiusgraden.

### Beispiel 1: 7-Trimethylsilylamino-3-jodomethylcephem(3)-4-carbonsäuretrimethylsilylester (Verbindung der Formel III)

### a) 7-Trimethylsilylamino-3-acetoxymethylcephem(3)-4-carbonsäuretrimethylsilylester (Formel IV):

Zu einer siedenden Suspension von 2,5 g 7-Aminocephaiosporansäure, 1,75 ml Trichlorsilan und 7,65 ml Hexamethyidisilazan in 100 ml trockenem Chloroform gibt man 45 mg Quadratsäure und kocht das Gemisch unter Stickstoffatmosphäre über Nacht unter Rückfluß. Die klare Lösung wird dann unter Vakuum zur Trockne eingedampft, wobei der 7-Trimethylsilylaminocephalosporansäuretrimethylsilylester in fester Form erhalten wird. Durch die extreme Hydrolysierbarkeit kann keine Schmelzpunktsbestimmung durchgeführt werden, die Charakterisierung erfolgt NMR-spektroskopisch.

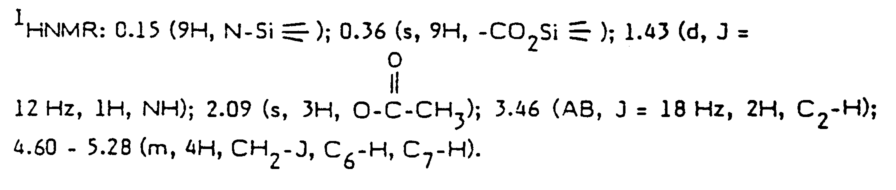

### b) 7-Trimethylsilylamino-3-jodomethylcephem(3)-4-carbonsäuretrimethylsilylester (Formel III):

Die N,O-bissilylierte 7-ACA (wie unter a) beschrieben hergestellt) wird in 125 ml trockenem Dichlormethan gelöst, die Lösung auf 0° gekühlt und 1,87 ml Trimethyljodsilan zugetropft. Man läßt die Lösung auf Raumtemperatur kommen und rührt 2 Stunden nach. Nach Abdampfen des Lösungsmittels im Vakuum erhält man die Titelverbindung in fester Form, die wegen der großen Hydrolyseempfindlichkeit nur NMR-spektroskopisch charakterisiert wurde.
¹HNMR(CDCl₃): 0.125 (b, 9H, N-Si〈̶ ); 0.38 (s, 9H, -CO₂Si〈̶ ); 1.55 (d, J = 12 Hz, 1H, NH); 3.50 (AB, J = 18 Hz, 2H, C₂H); 4.39 (AB, J = 9 Hz, 2H, -CH₂-J); 4.58 (d, d, J = 12 Hz, 4,5 Hz, 1H, C₇-H); 4.71 (d, J = 4,5 Hz, 1H, C₆-H).

Aus dem 7-Trimethylsilylamino-3-jodomethylcephem(3)-4-carbonsäuretri methylsilylester können beispielsweise folgende 3-substituierte Derivate der Formel II hergestellt werden:

### Beispiel 2: 7-Amino-3-pyridiniummethylcephem(3)-4-carbonsäure.Hydrojodid (Formel IIa):

Zu der Lösung der Titelverbindung des Beispiels 1 gibt man unter Eiskühlung 1,47 ml Pyridin und rührt die Lösung anschließend 2 Stunden bei Raumtemperatur. Durch Zugabe von 1,7 ml Methanol zur Lösung fällt man die rohe 7-Amino-3-pyridiniummethylcephem(3)-4-carbonsäure als Hydrojodid aus. Digerieren des Feststoffes in Wasser liefert die reine Titelverbindung. Fp: 145° (Zers.).
¹HNMR (D₂O, K₂CO₃): 3.38 (AB, J = 18 Hz, 2H, C₂-H); 4.80 (d, J = 5,8 Hz, 1H, C₇-H); 5.10 (d, J = 5,8 Hz, 1H, C₆-H); 5.43 (AB, J = 17 Hz, 2H, CH₂-N⁺〈̶); 8.0 - 8.2 (m, 2H, Pyridin 3H's); 8.41 - 8.63 (m, 1H, Pyridin-C₄-H); 8.93 (d, J = 6,3 Hz, 2H, Pyridin-2H's).

### Beispiel 3: 7-Amino-3-[5-(1,2,3-triazolyl)thiomethyl]cephem(3)-4-carbonsäure (Formel IIa):

Zu der Lösung der Titelverbindung des Beispiels 1 in Dichlormethan gibt man bei 0° 5 ml N,N-Dimethylformamid und 2,26 g 5-Mercapto-1,2,3-triazol.Na-salz und rührt die Lösung 2 Stunden bei Raumtemperatur weiter. Man gibt dann unter Eiskühlung 5 ml Methanol zu, wobei die Titelverbindung ausfällt. Man löst sie in 6 N HCl und stellt den pH mit konz. NaOH auf pH = 1, wobei die Titelverbindung wieder ausfällt. Zersetzungspunkt >190°.
NMR (DMSO; CF₃CO₂H): 3.28 (AB, J = 18 Hz, 2H); 3.95 (AB, J = 12,6 Hz, 2H); 4.76 (d, J = 5,7 Hz, 1H); 4.97 (d, J = 5,7 Hz, 1H); 7.9 (s, 1H).

### Beispiel 4: 7-β-Amino-3-(2-dehydrochinuclidinium)methylcephem(3)-4-carboxylat.Dihydrochlorid (Formel IIa):

2,22 g 7-Aminocephalosporansäure werden wie in Beispiel 1 beschrieben zu 7-Trimethylsilylamino-3-jodomethylcephem(3)-4-carbonsäuretrimethylsilylester umgesetzt. Zu dieser Lösung in Dichlormethan gibt man unter Eiskühlung 1 g 1-Aza-bicyclo[2.2.2]oct-2-en und rührt dann 2 Stunden bei Raumtemperatur nach. Man fällt das Rohprodukt durch Zugabe von 2 ml gesättigter etherischer HCl. Das Rohprodukt wird in verdünnter wäßriger HCl gelöst und mit Isopropanol ausgefällt. Man erhält ein farbloses Pulver. Zersetzung ab 140°.
¹HNMR (D₂O): 1.70 - 2.50 (m, 4H); 3.0 - 5.2 (m); 3.76 und 4.10 (AB, J = 19 Hz, 2H); 5.34 (d, J = 6 Hz, 1H); 5.53 (d, J = 6 Hz, 1H); 6.70 - 7.3 (m, 2H).

### Beispiel 5: 7-β-Amino-3-(N-methylpyrrolidinium)methylcephem(3)-4-carboxylat.Hydrochlorid (Formel IIa):

10,88 g 7-Aminocephalosporansäure werden wie beschrieben zu 7-Trimethylsilylamino-3-jodomethylcephem(3)-4-carbonsäuretrimethylsilylester umgesetzt. Zur Lösung dieser Verbindung in 120 ml Dichlormethan gibt man bei -60° langsam 4,15 ml N-Methylpyrrolidin und rührt 90 Minuten bei dieser Temperatur. Das Reaktionsgemisch wird dann auf 100 ml Ethanol, das mit etwas HCl-Gas versetzt ist, gegossen. Anschließend stellt man den pH der Suspension auf 2,5. Nach Stehen über Nacht wird der Festkörper isoliert. Er wird in 20 ml Wasser suspendiert und der pH wird mit konzentrierter HCl auf 0 gestellt. Man gibt dann 5 g Aktivkohle zu, rührt 5 Minuten und filtriert vom Ungelösten und der Aktivkohle ab. Der pH der farblosen wäßrigen Lösung wird dann mit 5 N NaOH auf 2,5 gestellt und zur Lösung werden langsam 200 ml Aceton zugegeben. Nach zweistündigem Stehen im Kühlschrank wird der Niederschlag über eine Nutsche isoliert und mit Aceton und Ether gewaschen. Nach Trocknen im Trockenschrank erhält man ein farbloses Pulver. Zersetzungspunkt: 115°. Ausbeute: 5,6 g (42 % d. Th.).
¹HNMR (CF₃CO₂D+D₂O): 5.48 (1H, d, J = 5,2 Hz, C₇-H); 5.31 (1H, d, J = 5,2 Hz, C₆-H); 4.73 (2H, AB, J = 13 Hz; CH₂-N-); 4.1-3.47 (6H, m, C₂-H und N-methyl-pyrrolidin-H); 3.15 (3H, s, CH₃-N-); 2.37 (4H, b, N-methylpyrrolidin-H).

Verbindungen der Formel I können folgendermaßen erhalten werden:

### Beispiel 6: (6R,7R)-7-[2-(2-Amino-4-thiazolyl]-(Z)-2-[(1-diphenylmethoxy carbonyl-1-methylethoxy)imino]acetamido-3-(1-pyridiniummethyl)cephem(3)--4-carbonsäurechlorid.Hydrochlorid (Formel I):

Zu einer siedenden Suspension von 2,5 g 7-ACA, 1,75 ml Trichlorsilan und 7,65 ml Hexamethyldisilazan in 100 ml trockenem Chloroform gibt man 45 mg Quadratsäure und kocht das Gemisch unter Stickstoffatmosphäre über Nacht unter Rückfluß. Die klare Lösung wird dann unter Vakuum zur Trockne eingedampft, wobei der 7-Trimethylsilylaminocephalosporansäuretrimethylsilylester in fester Form ausfällt. Man löst ihn in 125 ml trockenem Dichlormethan, kühlt auf 0° ab und tropft 1,87 ml Jodtrimethylsilan zu. Man läßt die Lösung auf Raumtemperatur kommen und rührt 2 Stunden nach. Zum entstandenen 7-Trimethylsilylamino-3-jodomethylcephem(3)-4-carbonsäuretrimethylsilylester gibt man 1,47 ml trockenes Pyridin und rührt 2 Stunden bei Raumtemperatur. Anschließend gibt man zu dieser Lösung 5,41 g 2-(2-Amino-4-thiazolyl)-(Z)-2-[(1-diphenylmethoxycarbonyl-1-methylethoxy)imino]-thioessigsäure-S-benzothiazol-2-ylester und rührt das Gemisch 2 Tage bei Raumtemperatur. Das Reaktionsgemisch wird dann in eine 50° warme Lösung von 4,5 ml konzentrierter Salzsäure in 500 ml Isopropanol eingetragen, wobei die Titelverbindung ausfällt. Sie wird abgesaugt und mit Isopropanol gewaschen. Nach Trocknung und Umkristallisation aus wäßriger HCl/Isopropanol erhält man die Titelverbindung als gelblich gefärbtes Produkt.
Fp.: Zersetzung ab 160°
¹H-NMR (DMSO-d₆): 9.84 (1H, d, -N-H, J = 8 Hz); 9.20, 8.67 und 8.23 (5H, Pyridinium-H); 7.28 (10H, Phenyl-H); 6.98 (1H, S, Thiazolyl-H); 6.79 (1H, S, CH-Ph₂); 5.95 (1H, dd, H₇,. J = 8 Hz, J = 5 Hz); 5.72 (2H, CH₂-Pyridin); 5.27 (1H, d, H₆, J = 5 Hz); 3.57 (2H, H₂ und H₂'); 1.63 (6H, -C(CH₃)₂).

### Beispiel 7: 7-[α-(Ethoxycarbonylmethoxy)imino-1H-pyrazol-3-yl]acetamido-3-[(1-methyl-1H-tetrazol-5-yl)thiomethyl]cephem(3)-4-carbonsäure.Ethanolsolvat (Formel I):

Zu einer Lösung von 3,82 g 7-Trimethylsilylamino-3-jodomethyl-3-cephem-4-carbonsäuretrimethylsilylester (hergestellt wie in Beispiel 1 oder 6 beschrieben) in 125 ml trockenem Dichlormethan gibt man 5 ml Dimethylformamid und 1,4 g 1-Methyl-5-mercapto-1,2,3,4-tetrazol.Natriumsalz und rührt die Lösung 2 Stunden bei Raumtemperatur. Anschließend gibt man 4,16 g kristallinen (Methoxycarbonylmethoximino)-1H-pyrazol-3-yl-essigsäure-mercaptobenzthiazolylester zu und rührt das Reaktionsgemisch über Nacht bei Raumtemperatur. Der Ansatz wird nun in 350 ml auf 55° vorgewärmtes Ethanol eingerührt und langsam auf 30° abgekühlt. Durch Animpfen mit der Titelverbindung fällt die Titelverbindung in kristalliner Form aus, sie wird abfiltriert und getrocknet.
Fp.: 119 - 121°.

### Beispiel 8: 7-β-(2-Thienylacetamido)-3-pyridiniummethylceohem(3)-4-carbonsäure.Nitrat (Formel I):

Zu einer Lösung von 3,82 g 7-Trimethylsilylamino-3-jodomethylcephem(3)-4-carbonsäuretrimethylsilylester (hergestellt wie in Beispiel 1 oder 6 beschrieben) in 125 ml trockenem Dichlormethan gibt man 1,47 ml trockenes Pyridin und rührt 2 Stunden bei Raumtemperatur. Anschließend gibt man zu dieser Lösung eine Lösung von 3,2 g Thienylessigsäure-S-mercaptobenzthiazolylester in 20 ml Dichlormethan (hergestellt in situ aus 1,56 g Thiophenessigsäure, 4,47 g Bis(benzothiazol-2-yl)disulfid und 3,53 g Triphenylphosphin in Dichlormethan) und rührt das Gemisch 30 Stunden bei Raumtemperatur. Durch Zugabe von 5 ml Methanol fällt man die rohe Titelverbindung als Hydrojodid. Diese wird in 5 % Bicarbonatlösung gelöst, von Unlöslichem abfiltriert, und durch Zugabe von verdünnter Salpetersäure kristallisiert die Titelverbindung bei einem pH von 1,8 als Nitrat aus.
Fp.: 151 - 152° (Zers.).

## Patentansprüche (Patentansprüche für folgende(n) Vertragsstaat(en): BE, CH, DE, FR, GB, IT, LU, NL, SE)

1. Cephalosporinderivat der Formel 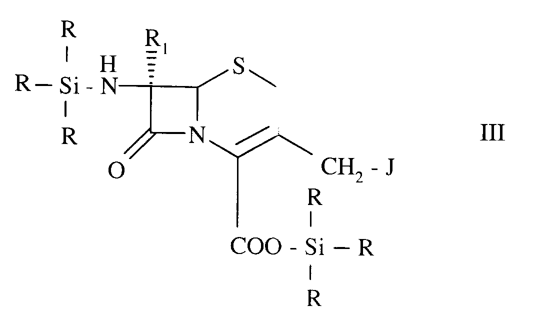 worin R für eine niedere Alkylgruppe und R₁ für Wasserstoff oder die Methoxygruppe stehen.

2. Verwendung eines Cephalosporinderivates der Formel III nach Anspruch 1 als Zwischenprodukt für die Herstellung eines Cephalosporinderivats.

3. Verwendung nach Anspruch 2, dadurch gekennzeichnet, dass das Cephalosporinderivat der Formel III durch Umsetzung einer Verbindung der Formel 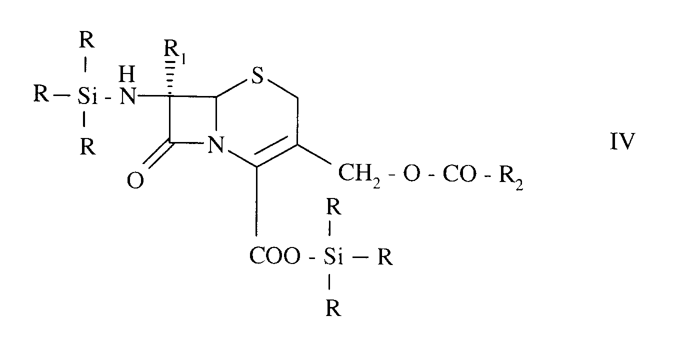 worin R und R₁ die in Anspruch 1 angegebene Bedeutung haben und R₂ für die Methyl- oder die Aminogruppe steht, mit einem Trialkyljodsilan hergestellt ist.

4. Verwendung nach Anspruch 3, dadurch gekennzeichnet, dass das Trialkyljodsilan Trimethyljodsilan ist.

5. Verwendung nach einem der Ansprüche 3 oder 4, dadurch gekennzeichnet, dass man 1,1 bis 1,5 Äquivalente Trialkyljodsilan pro Äquivalent Cephalosporinderivat der Formel IV einsetzt.

6. Verfahren zur Herstellung eines Cephalosporinderivates der Fromel 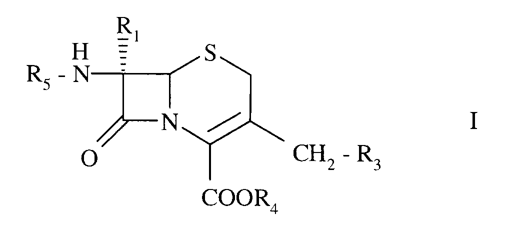 worin
R₁ Wasserstoff oder Methoxy,
R₃ den Rest eines Nukleophils,
R₄ Wasserstoff, Alkali oder eine negative Ladung, oder OR₄ Halogen und
R₅ eine in der Cephalosporinchemie übliche Acylgruppe bedeuten,
dadurch gekennzeichnet, dass ein Cephalosporinderivat der Formel III nach Anspruch 1 mit einem gewünschten Nucleophil umgesetzt wird und das dabei erhaltene Derivat, das in 3-Stellung durch den Rest des Nucleophils substituiert ist, entweder durch Abspaltung der Silylschutzgruppen entschützt oder mit einem Acylierungsmittel in 7-Stellung acyliert und durch Abspaltung der Silylschutzgruppen entschützt wird.

7. Verfahren nach Anspruch 6, dadurch gekennzeichnet, dass das Cephalosporanderivat der Formel III gemäss einem der Ansprüche 3 bis 5 hergestellt ist.

## Patentansprüche (Patentansprüche für folgende(n) Vertragsstaat(en): AT)

1. Verwendung eines Cephalosporinderivats der Formel 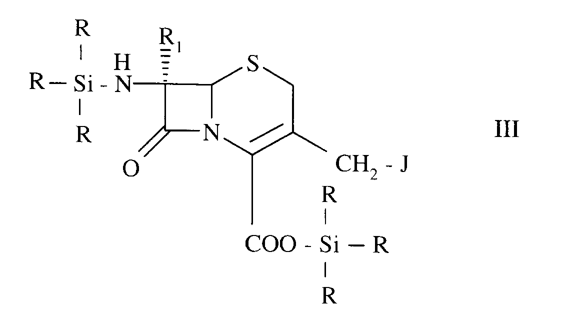 worin R für eine niedere Alkylgruppe und R₁ für Wasserstoff oder die Methoxygruppe stehen, als Zwischenprodukt für die Herstellung eines Cephalosporinderivats.

2. Verwendung nach Anspruch 1, dadurch gekennzeichnet, dass das Cephalosporinderivat der Formel III durch Umsetzung einer Verbindung der Formel 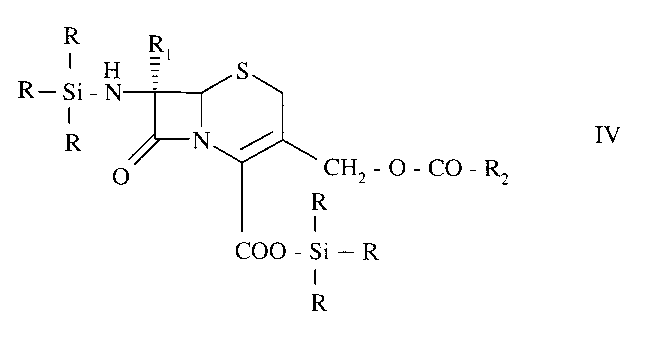 worin R und R₁ die im Anspruch 1 angegebene Bedeutung haben und R₂ für die Methyl- oder die Aminogruppe steht, mit einem Trialkyljodsilan hergestellt ist.

3. Verwendung nach Anspruch 2, dadurch gekennzeichnet, dass das Trialkyljodsilan Trimethyljodsilan ist.

4. Verwendung nach einem der Ansprüche 2 oder 3, dadurch gekennzeichnet, dass man 1,1 bis 1,5 Äquivalente Trialkyljodsilan pro Äquivalent Cephalosporinderivat der Formel IV einsetzt.

5. Verfahren zur Herstellung eines Cephalosporinderivates der Fromel 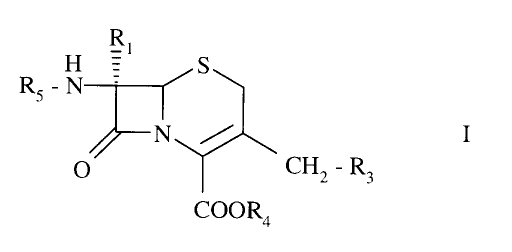 worin
R₁ Wasserstoff oder Methoxy,
R₃ den Rest eines Nukleophils,
R₄ Wasserstoff, Alkali oder eine negative Ladung, oder OR₄ Halogen und
R₅ eine in der Cephalosporinchemie übliche Acylgruppe bedeuten,
dadurch gekennzeichnet, dass ein Cephalosporinderivat der Formel III nach Anspruch 1 mit einem gewünschten Nucleophil umgesetzt wird und das dabei erhaltene Derivat, das in 3-Stellung durch den Rest des Nucleophils substituiert ist, entweder durch Abspaltung der Silylschutzgruppen entschützt oder mit einem Acylierungsmittel in 7-Stellung acyliert und durch Abspaltung der Silylschutzgruppen entschützt wird.

6. Verfahren nach Anspruch 5, dadurch gekennzeichnet, dass das Cephalosporinderivat der Formel III gemäss einem der Ansprüche 2 bis 4 hergestellt ist.

## Claims (Claims for the following Contracting State(s): BE, CH, DE, FR, GB, IT, LU, NL, SE)

1. A cephalosporin derivative of formula 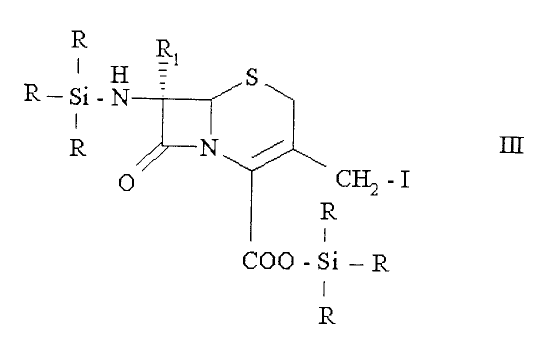 wherein R denotes a lower alkyl group and R₁ denotes hydrogen or the methoxy group.

2. Use of a cephalosporin derivative of formula III according to claim 1 as an intermediate in the production of a cephalosporin derivative.

3. Use according to claim 2, characterized in that the cephalosporin derivative of formula III is obtained by reaction of a compound of formula 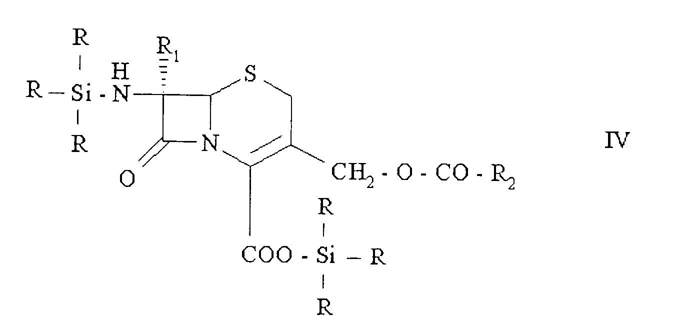 wherein R and R₁ are as defined in claim 1 and R₂ denotes the methyl or the amine group, with a trialkyliodosilane.

4. Use according to claim 3, characterized in that the trialkyliodosilane is trimethyliodosilane.

5. Use according to any one of claims 3 or 4 characterized in that 1.1 to 1.5 equivalents of trialkyliodosilane are used per equivalent of cephalosporin derivative of formula IV.

6. Process for the production of a cephalosporin derivative of formula 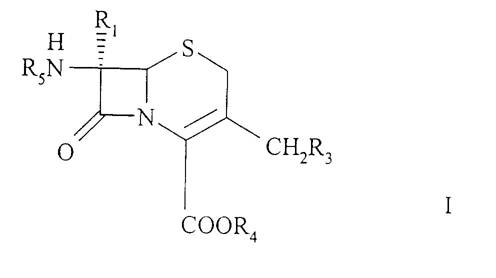 wherein
R₁ is hydrogen or methoxy,
R₃ is the residue of a nucleophile,
R₄ is hydrogen, alkali or a negative charge, or OR₄ is halogen and
R₅ is an acyl group conventional in cephalosporin chemistry,
characterised in that a cephalosporin derivative of formula III according to claim 1 is reacted with a desired nucleophile and the derivative obtained which is substituted in position 3 by the residue of the nucleophile is either deprotected by splitting off the silyl protecting groups, or acylated with an acylating agent in position 7 and deprotected by splitting off the silyl protecting groups.

7. Process according to claim 6, characterised in that the cephalosporin derivative of formula III is obtained according to any one of claims 3 to 5.

## Claims (Claims for the following Contracting State(s): AT)

1. Use of a cephalosporin derivative of formula 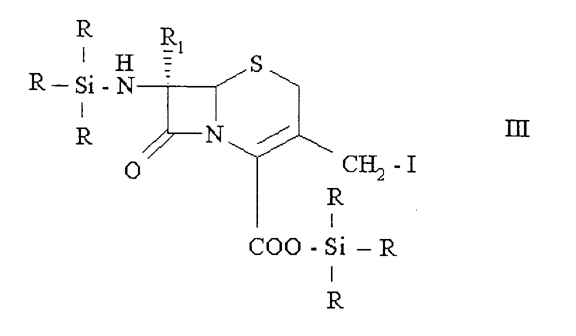 wherein R denotes a lower alkyl group and R₁ denotes hydrogen or the methoxy group, as an intermediate in the production of a cephalosporin derivative.

2. Use according to claim 1, characterized in that the cephalosporin derivative of formula III is obtained by reaction of a compound of formula 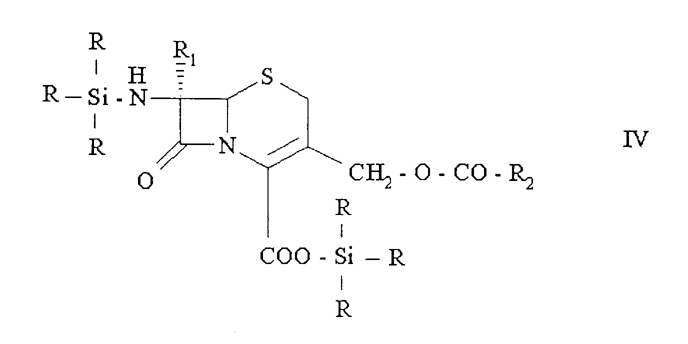 wherein R and R₁ are as defined in claim 1 and R₂ denotes the methyl or the amine group, with a trialkyliodosilane.

3. Use according to claim 2, characterized in that the trialkyliodosilane is trimethyliodosilane.

4. Use according to any one of claims 2 or 3 characterized in that 1.1 to 1.5 equivalents of trialkyliodosilane are used per equivalent of cephalosporin derivative of formula IV.

5. Process for the production of a cephalosporin derivative of formula 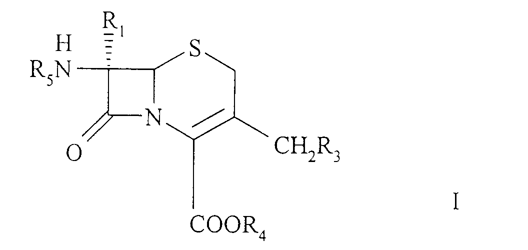 wherein
R₁ is hydrogen or methoxy,
R₃ is the residue of a nucleophile,
R₄ is hydrogen, alkali or a negative charge, or OR₄ is halogen and
R₅ is an acyl group conventional in cephalosporin chemistry,
characterised in that a cephalosporin derivative of formula III according to claim 1 is reacted with a desired nucleophile and the derivative obtained which is substituted in position 3 by the residue of the nucleophile is either deprotected by splitting off the silyl protecting groups, or acylated with an acylating agent in position 7 and deprotected by splitting off the silyl protecting groups.

6. Process according to claim 5, characterised in that the cephalosporin derivative of formula III is obtained according to any one of claims 2 to 4.

## Revendications (Revendications pour l'(les) Etat(s) contractant(s) suivant(s): BE, CH, DE, FR, GB, IT, LU, NL, SE)

1. Un dérivé de la céphalosporine de formule 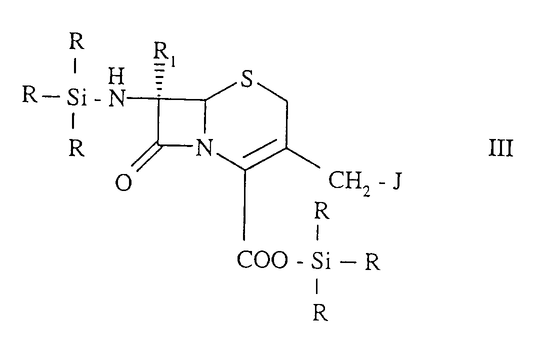 où R signifie un groupe alkyle inférieur et R₁ signifie l'hydrogène ou un groupe méthoxy.

2. Utilisation d'un dérivé de la céphalosporine de formule III selon la revendication 1, comme produit intermédiaire pour la préparation d'un dérivé de la céphalosporine.

3. Utilisation selon la revendication 2, caractérisée en ce qu'on prépare le dérivé de la céphalosporine de formule III par réaction d'un composé de formule 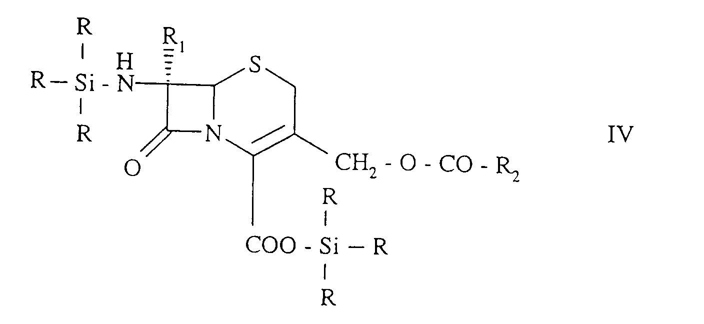 où R et R₁ ont la signification donnée à la revendication 1 et R₂ signifie un groupe méthyle ou amino, avec un trialkyliodosilane.

4. Utilisation selon la revendication 3, caractérisée en ce que le trialkyliodosilane est le triméthyliodosilane.

5. Utilisation selon l'une des revendications 3 ou 4, caractérisée en ce qu'on utilise 1,1 à 1,5 équivalent de trialkyliodosilane par équivalent du dérivé de la céphalosporine de formule IV.

6. Procédé de préparation d'un dérivé de la céphalosporine de formule 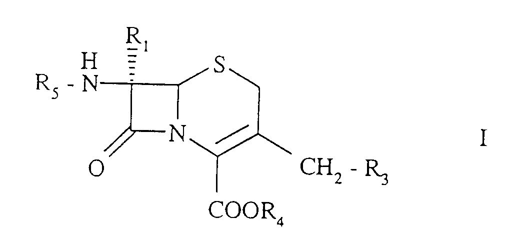 où
R₁ signifie l'hydrogène ou un groupe méthoxy,
R₃ signifie le reste d'un nucléophile,
R₄ signifie l'hydrogène, un métal alcalin ou une charge négative, ou bien OR₄ signifie un halogène et
R₅ signifie un groupe acyle habituel dans la chimie des céphalosporines,
caractérisé en ce qu'on fait réagir un dérivé de la céphalosporine de formule III selon la revendication 1 avec un nucléophile désiré et le dérivé obtenu, qui est substitué en position 3 par le reste du nucléophile, est déprotégé par élimination des groupes protecteurs du groupe silyle ou bien acylé avec un agent d'acylation en position 7 et déprotégé par élimination des groupes protecteurs du groupe silyle.

7. Procédé selon la revendication 6, caractérisé en ce que le dérivé de la céphalosporine de formule III est préparé selon l'une des revendications 3 à 5.

## Revendications (Revendications pour l'(les) Etat(s) contractant(s) suivant(s): AT)

1. Utilisation d'un dérivé de la céphalosporine de formule 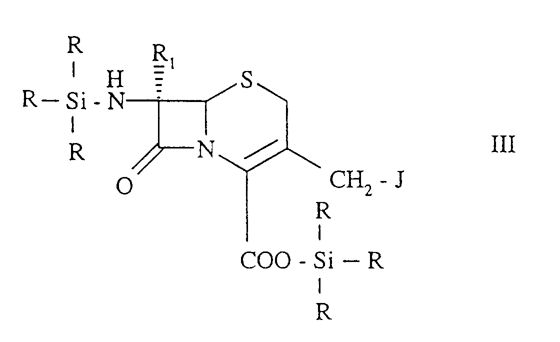 où R signifie un groupe alkyle inférieur et R₁ signifie l'hydrogène ou un groupe méthoxy, comme produit intermédiaire pour la préparation d'un dérivé de la céphalosporine.

2. Utilisation selon la revendication 1, caractérisée en ce qu'on prépare le dérivé de la céphalosporine de formule III par réaction d'un composé de formule 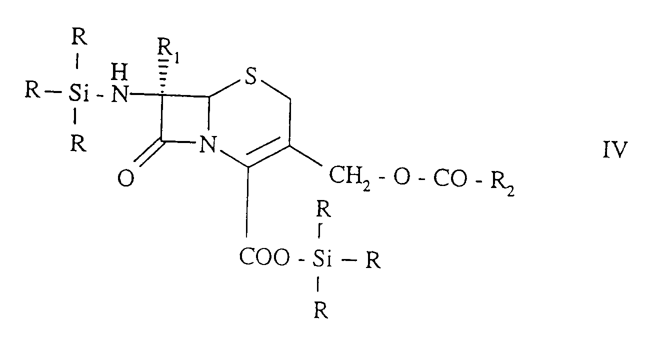 où R et R₁ ont la signification donnée à la revendication 1 et R₂ signifie un groupe méthyle ou amino, avec un trialkyliodosilane.

3. Utilisation selon la revendication 2, caractérisée en ce que le trialkyliodosilane est le triméthyliodosilane.

4. Utilisation selon l'une des revendications 2 ou 3, caractérisée en ce qu'on utilise de 1,1 à 1,5 équivalent de trialkyliodosilane par équivalent de dérivé de la céphalosporine de formule IV.

5. Procédé de préparation d'un dérivé de la céphalosporine de formule 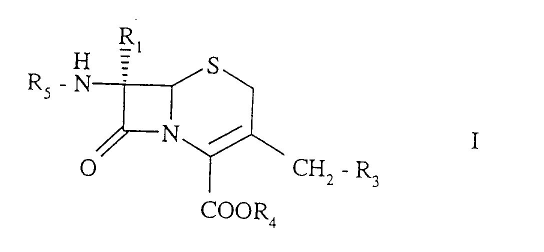 où
R₁ signifie l'hydrogène ou un groupe méthoxy,
R₃ signifie le reste d'un nucléophile,
R₄ signifie l'hydrogène, un métal alcalin ou une charge négative, ou bien OR₄ signifie un halogène et
R₅ signifie un groupe acyle habituel dans la chimie des céphalosporines,
caractérisé en ce qu'on fait réagir un dérivé de la céphalosporine de formule III selon la revendication 1 avec un nucléophile désiré et le dérivé obtenu, qui est substitué en position 3 par le reste du nucléophile, est déprotégé par élimination des groupes protecteurs du groupe silyle ou bien acylé avec un agent d'acylation en position 7 et déprotégé par élimination des groupes protecteurs du groupe silyle.

6. Procédé selon la revendication 5, caractérisé en ce que le dérivé de la céphalosporine de formule III est préparé selon l'une des revendications 2 à 4.
